(19) Europäisches Patentamt
European Patent Office
Office européen des brevets

(11) **EP 2 332 964 B1**

(12) **EUROPÄISCHE PATENTSCHRIFT**

(45) Veröffentlichungstag und Bekanntmachung des Hinweises auf die Patenterteilung:
**20.04.2016 Patentblatt 2016/16**

(51) Int Cl.:
**C07K 7/06** *(2006.01)* **A61K 38/08** *(2006.01)*

(21) Anmeldenummer: **10010931.3**

(22) Anmeldetag: **18.12.2002**

(54) **Apoptotisch wirksame Peptide**

APOPTOTICALLY ACTIVE PEPTIDES

PEPTIDES AYANT UN EFFET SUR L'APOPTOSE

(84) Benannte Vertragsstaaten:
**AT BE BG CH CY CZ DE DK EE ES FI FR GB GR IE IT LI LU MC NL PT SE SI SK TR**

(30) Priorität: **20.12.2001 DE 10163130**

(43) Veröffentlichungstag der Anmeldung:
**15.06.2011 Patentblatt 2011/24**

(62) Dokumentnummer(n) der früheren Anmeldung(en) nach Art. 76 EPÜ:
**02805332.0 / 1 458 748**

(73) Patentinhaber: **CytoTools AG**
**64285 Darmstadt (DE)**

(72) Erfinder:
• **Freyberg, Mark**
**64285 Darmstadt (DE)**
• **Kaiser, Dirk**
**64859 Eppertshausen (DE)**
• **Friedl, Peter**
**64823 Groß-Umstadt (DE)**

(74) Vertreter: **Schultheiss, Jürgen**
**Hauptstraße 17-19**
**Gebäude 6301A**
**55120 Mainz (DE)**

(56) Entgegenhaltungen:
WO-A-00/45856    WO-A-00/47102
WO-A-96/11942    WO-A-96/17059
WO-A1-98/58541    WO-A1-99/54464
US-A- 5 399 667

• **DATABASE EMBL [Online] 1. November 2001 (2001-11-01), SKEIKY Y. A. W. ET AL.: "Streptomyces lividans ebrC repressor (ebrS) and multidrug resistance efflux protein (ebrC) genes, complete cds", XP002248159, Database accession no. AAU58153**
• **DATABASE GENBANK [Online] READ T. ET AL.: "Bacillus anthracis str. Ames section 1 of 18 of the complete genome", XP002248152, Database accession no. ae017024**
• **DATABASE GENBANK [Online] 25. Mai 2000 (2000-05-25), TETTELIN H. ET AL.: "Neisseria meningitidis serogroup B strain MC58 section 157 of 206 of the complete genome", XP002248153, Database accession no. AE002515**
• **DATABASE GENBANK [Online] 25. Mai 2000 (2000-05-25), TETTELIN H. ET AL.: "Neisseria meningitidis serogroup B strain MC58 section 36 to 206 of the complete genome", XP002248157, Database accession no. AE002394**
• **DATABASE GENBANK [Online] 3. November 2001 (2001-11-03), LEE L. F. ET AL.: "Streptomyces lividans ebrC repressor (ebrS) and multidrug resistance efflux protein (ebrC) genes, complete cds", XP002248158, Database accession no. AY043331**
• **DATABASE GENBANK [Online] 11. März 1998 (1998-03-11), DUBOIS E. ET AL.: "S. cerevisiae chomosome II reading frame ORF YBR222c", XP002248154, Database accession no. Z36091**
• **DATABASE GENBANK [Online] 29. Mai 1998 (1998-05-29), FLEISCHMANN R. D. ET AL.: "Haemophilus influenzae Rd section 148 of 163 of the complete genome", XP002248148, Database accession no. U32833**

- DATABASE GENBANK [Online] 19. September 1995 (1995-09-19), FRIEDBERG D. ET AL.: "Salmonella typhimurium leucine-responsive regulatory protein (Lrp) gene, complete cds", XP002248149, Database accession no. U02273
- DATABASE GENBANK [Online] 3. Juni 2000 (2000-06-03), NAKAJIMA M. ET AL.: "Pseudomonas syringae plasmid pPaCu1 ORFA, ORFB, ORFC, ORFD genes, complete cds", XP002248156, Database accession no. AB033419
- DATABASE GENBANK [Online] 17. Dezember 2001 (2001-12-17), STAPLETON M. ET AL.: "Drosophila melanogaster LD41918 full length cDNA", XP002248151, Database accession no. AY069650
- DATABASE GENBANK [Online] 1. Juni 2000 (2000-06-01), HARTMANN E. & GOERLICH D.: "Drosophila melanogaster Ran binding protein 11 (Ranbp11) mRNA, complete cds", XP002248150, Database accession no. AF245515
- DATABASE GENBANK [Online] 17. März 1994 (1994-03-17), LEE Y. A. ET AL.: "Xanthomonas campestris juglandis copper-resistance genes, complete cds", XP002248155, Database accession no. L19222
- CARRON J A ET AL: "A CD36-binding peptide from thrombospondin-1 can stimulate resorption by osteoclasts in vitro.", BIOCHEMICAL AND BIOPHYSICAL RESEARCH COMMUNICATIONS, Bd. 270, Nr. 3, 21. April 2000 (2000-04-21), Seiten 1124-1127, XP002248144, ISSN: 0006-291X
- GAO AI-GUO ET AL: "Integrin-associated protein is a receptor for the C-terminal domain of thrombospondin.", JOURNAL OF BIOLOGICAL CHEMISTRY, Bd. 271, Nr. 1, 1996, Seiten 21-24, XP002248145, ISSN: 0021-9258
- GAO A-G ET AL: "THROMBOSPONDIN MODULATES ALPHAVBETA3 FUNCTION THROUGH INTEGRIN-ASSOCIATED PROTEIN", THE JOURNAL OF CELL BIOLOGY, ROCKEFELLER UNIVERSITY PRESS, US, Bd. 135, Nr. 2, Oktober 1996 (1996-10), Seiten 533-544, XP000990435, ISSN: 0021-9525
- KOSFELD MINH D ET AL: "Identification of a new cell adhesion motif in two homologous peptides from the COOH-terminal cell binding domain of human thrombospondin.", JOURNAL OF BIOLOGICAL CHEMISTRY, Bd. 268, Nr. 12, 1993, Seiten 8808-8814, XP002248146, ISSN: 0021-9258
- JIMENEZ BENILDE ET AL: "Signals leading to apoptosis-dependent inhibition of neovascularization by thrombospondin-1.", NATURE MEDICINE., Bd. 6, Nr. 1, Januar 2000 (2000-01), Seiten 41-48, XP002248147, ISSN: 1078-8956

SEQUENZPROTOKOLL

<110> CytoTools GmbH
<120> Apoptotisch wirksame Peptide
<130> C 688wo
<160> 17
<170> PatentIn version 3.1

<210> 1
<211> 6
<212> PRT
<213> Künstliche Sequenz

<220>
<223> Synthetisches Peptid

<400> 1

```
                              Arg Ala Tyr Val Val Met
                              1                   5
```

<210> 2
<211> 6
<212> PRT
<213> Künstliche Sequenz

<220>
<223> Synthetisches Peptid

<400> 2

```
                              Arg Trp Tyr Val Val Met
                              1                   5
```

<210> 3
<211> 6
<212> PRT
<213> Künstliche Sequenz

<220>
<223> Synthetisches Peptid

<400> 3

```
                              Arg Tyr Tyr Val Val Met
                              1                   5
```

<210> 4
<211> 6
<212> PRT
<213> Künstliche Sequenz

<220>
<223> Synthetisches Peptid

<400> 4

```
                              Arg Glu Tyr Val Val Met
                              1                   5
```

<210> 5
<211> 10
<212> PRT
<213> Künstliche Sequenz

<220>
<223> Synthetisches Peptid

<400> 5

```
                    Lys Arg Ala Tyr Val Val Met Trp Lys Lys
                    1               5                   10
```

<210> 6

<211> 10
<212> PRT
<213> Künstliche Sequenz

<220>
<223> Synthetisches Peptid

<400> 6

```
Lys Arg Glu Tyr Val Val Met Trp Lys Lys
1               5                   10
```

<210> 7
<211> 6
<212> PRT
<213> Künstliche Sequenz

<220>
<223> Synthetisches Peptid

<400> 7

```
Arg Gly Tyr Val Val Met
1               5
```

<210> 8
<211> 6
<212> PRT
<213> Künstliche Sequenz

<220>
<223> Synthetisches Peptid

<400> 8

```
Arg Met Tyr Val Val Met
1               5
```

<210> 9
<211> 6
<212> PRT
<213> Künstliche Sequenz

<220>
<223> Synthetisches Peptid

<400> 9

```
Arg Thr Tyr Val Val Met
1               5
```

<210> 10
<211> 6
<212> PRT

<213> Künstliche Sequenz

<220>
<223> Synthetisches Peptid

<400> 10

```
Arg Asn Tyr Val Val Met
1               5
```

<210> 11
<211> 6
<212> PRT
<213> Künstliche Sequenz

<220>
<223> Synthetisches Peptid

<400> 11

```
Arg Asp Tyr Val Val Met
1               5
```

<210> 12
<211> 6
<212> PRT
<213> Künstliche Sequenz

<220>
<223> Synthetisches Peptid

<400> 12

```
Met Val Val Tyr Phe Arg
1             5
```

<210> 13
<211> 6
<212> PRT
<213> Künstliche Sequenz

<220>
<223> Synthetisches Peptid

<400> 13

```
Arg Ala Tyr Val Val Ala
1               5
```

<210> 14
<211> 6
<212> PRT
<213> Künstliche Sequenz

<220>
<223> Synthetisches Peptid

<400> 14

```
                                    Arg Leu Tyr Val Val Met
                                    1               5
```

<210> 15
<211> 6
<212> PRT
<213> Künstliche Sequenz

<220>
<223> Synthetisches Peptid

<400> 15

```
                                    Arg Pro Tyr Val Val Met
                                    1               5
```

<210> 16
<211> 6
<212> PRT
<213> Künstliche Sequenz

<220>
<223> Synthetisches Peptid

<400> 16

```
                                    Arg Ser Tyr Val Val Met
                                    1               5
```

<210> 17
<211> 6
<212> PRT
<213> Künstliche Sequenz

<220>
<223> Synthetisches Peptid

<400> 17

```
                                    Arg Cys Tyr Val Val Met
                                    1               5
```

<210> 18
<211> 6
<212> PRT
<213> Künstliche Sequenz aus D-Aminosäuren

<220>
<223> Synthetisches Peptid

<400> 18

```
met val val tyr phe arg
1               5
```

<210> 19
<211> 6
<212> PRT
<213> Künstliche Sequenz aus D-Aminosäuren

<220>
<223> Synthetisches Peptid

<400> 19

```
met val val tyr ala arg
1               5
```

**Beschreibung**

Die vorliegende Erfindung betrifft apoptotisch wirksame Peptide sowie deren Verwendung zur Herstellung von Medikamenten.

Apoptose ist ein genetisch kodiertes Selbstmordprogramm, welches in eukaryontischen Zellen unter bestimmten physiologischen oder pathologischen Bedingungen induziert wird. Die Induktion der Apoptose muss außerordentlich präzise reguliert sein, denn eine Hyperaktivität kann zu degenerativen Erkrankungen führen. Auf der anderen Seite kann eine verringerte Apoptose-Induktion zur Tumorprogression beitragen.

Verschiedene niedermolekulare Induktoren der Apoptose wurden bereits beschrieben. Eine wichtige Klasse sind Tumorcytostatika. Auf welche Weise diese Cytostatika oder andere Substanzen Apoptose induzieren können, ist in den meisten Fällen jedoch unbekannt.

Die Induktion von Apoptose kann beispielsweise über eine Reihe von sog. Todesrezeptoren, d. h. Rezeptoren, die eine "Death Domain" (DD) enthalten, wie CD95, TNF-RI, DR3, DR4 oder DR5, erfolgen, die nach Bindung ihrer Liganden Apoptose-Signalwege induzieren. Beispielsweise interagiert nach Bindung des CD95-Liganden der CD95-Rezeptor mit dem Adapterprotein FADD/MORT1, wodurch das "Recruitment" und die Aktivierung der Protease FLICE/Caspase-8, am DISC "Death Inducing Signalling Complex" induziert werden. FADD und FLICE enthalten jeweils "Death Effector Domains" (DED). Die Induktion der Apoptose über diese Apoptose-Signalwege ist von außen beispielsweise durch die Gabe von Zellgiften (cytotoxischen Substanzen), Bestrahlung, Viren, Entzug von Wachstumsfaktoren oder mechanische Zellverletzung möglich. Diese Möglichkeiten der Apoptose-Induktion sind allerdings von bestimmten Nachteilen begleitet. So führt die Gabe von Zellgiften, wie Cytostatika, oder die Bestrahlung bei Krebszellen zur Resistenzentwicklung und darüber hinaus zu einer Schädigung normaler Zellen, bei denen eigentlich keine Apoptose ausgelöst werden sollte.

Im Allgemeinen wird z.B. die Induktion der Apoptose vorgeschlagen zur Behandlung von Krebs, zur Verhinderung von angiogenetischen Vorgängen etc. Obwohl hier bereits Induktoren beschrieben wurden, weisen diese noch immer eine Reihe von Nachteilen auf. Beispielsweise erzeugen Cytostatika schwere Nebenwirkungen.

Es besteht daher ein hoher Bedarf an Substanzen, die die Apoptose positiv beeinflussen. Weiterhin besteht hoher Bedarf an pharmazeutischen Formulierungen, die solche Substanzen enthalten, und die zur Behandlung von Zuständen eingesetzt werden können, bei denen die Induktion der Apoptose angezeigt ist, insbesondere zur Behandlung von Krebs. Diese Substanzen sollten dabei möglichst von geringem Molekulargewicht und/oder kleine Peptide sein, um eine gute Bioverfügbarkeit zu gewährleisten.

Aufgabe der vorliegenden Erfindung war es daher, apoptotisch wirksame Substanzen, bevorzugt Peptide, zur Verfügung zu stellen. Eine weitere Aufgabe der vorliegenden Erfindung war es, pharmazeutische Zubereitungen zur Verfügung zu stellen, mit denen Krankheiten wie Krebs behandelt werden können, bei denen die Induktion von Apoptose angezeigt ist.

Diese und weitere, nicht explizit genannte Aufgaben, die sich aber aus der vorangegangenen Würdigung des Stands der Technik ohne weiteres ergeben, werden durch die in den Ansprüchen definierten Ausführungsbeispiele der vorliegenden Erfindung gelöst.

Insbesondere wird diese Aufgabe durch zur Verfügungstellen von Substanzen mit allen Merkmalen des Anspruchs 1.

Für die Zwecke der vorliegenden Erfindung wird der international übliche Einbuchstabencode für Aminosäuren verwendet, A steht also für Alanin (Ala), C für Cystein (Cys), D für Asparaginsäure (Asp), E für Glutaminsäure (Glu), F für Phenylalanin (Phe), G für Glycin (Gly), L für Leucin (Leu), M für Methionin (Met), N für Asparagin (Asn), P für Prolin (Pro), R für Arginin (Arg), S für Serin (Ser), T für Threonin (Thr), V für Valin (Val), W für Tryptophan (Trp) und Y für Tyrosin (Tyr). L-Aminosäuren werden hierbei durch Großbuchstaben und D-Aminosäuren durch die Verwendung von Kleinbuchstaben symbolisiert.

Unter einem weiteren besonders bevorzugten Gesichtspunkt betrifft die vorliegende Erfindung auch Apoptose induzierende Substanzen, bevorzugt Proteine oder Peptide, die eine der unter SEQ ID NO 12, SEQ ID NO 18 und SEQ ID NO 19 gezeigten Peptidsequenzen umfassen, oder deren entsprechende pharmazeutisch annehmbare Salze.

Weiterhin betrifft die vorliegende Erfindung die Verwendung der erfindungsgemäßen Peptide umfassend mindestens eine der in SEQ ID NO 12, SEQ ID NO 18 und SEQ ID NO 19 dargestellten Aminosäuresequenz zur Herstellung von Medikamenten, insbesondere zur Herstellung von Medikamenten bei denen ein Absterben von Zellen herbeigeführt werden soll wie beispielsweise zur Behandlung von Krebs.

Als ganz besonders bevorzugte Ausführungsbeispiele der vorliegenden Erfindung werden folgende Peptide/Peptidsequenzen zur Verfügung gestellt:

    12. M-V-V-Y-F-R (SEQ ID NO 12)
    18. m-v-v-y-f (SEQ ID NO 18)
    19. m-v-v-y-a-r (SEQ ID NO 19)

Für die Zwecke der vorliegenden Erfindung wird unter dem Begriff "Peptid" eine Substanz verstanden, die aus einer Kette von 2 oder mehr, durch Peptidbindungen verbundenen Aminosäuren, besteht. Insbesondere weisen erfindungsgemäße apoptotisch wirksame Peptide eine Kettenlänge von < 100 Aminosäuren, bevorzugt <75, besonders bevorzugt von <50, ganz besonders bevorzugt von <25 und am meisten bevorzugt von <15 Aminosäuren auf.

Besonders bevorzugt werden daher für die erfindungsgemäßen Zwecke Peptide, die eine der SEQ ID Nos12, 18 und 19 umfassen und entweder am N-terminalen und/oder am C-terminalen Ende 1 bis 3 zusätzliche Aminosäuren umfassen.

Für die Zwecke der vorliegenden Erfindung wird unter dem Begriff "Protein" eine Substanz verstanden, in der mehrere "Peptide" beispielsweise durch molekulare Bindungen wie Disulfidbrücken oder durch Salzbrücken miteinander verbunden sind. Diese Definition umfasst gleichermaßen native Proteine als auch zumindest teilweise "künstliche" Proteine, wobei solche "künstlichen" Proteine z.B. durch Anfügen von chemischen Resten an die Aminosäurekette verändert sein können, die bei nativen Proteinen normalerweise nicht vorkommen.

Für die Zwecke der vorliegenden Erfindung wird unter "apoptotisch wirksam" verstanden, dass der Zusatz der entsprechenden Substanz in das in den Beispielen 5 und 6 gezeigte Testsystem einen positiven oder negativen Inhibitionsindex erzeugt. Insbesondere werden hiefür Endothelzellen, besonders bevorzugt HUVEC, in Medium kultiviert, welches 1$\mu$g TSP-1 pro ml enthält. Die prozentualen Apoptosewerte dieser Positivkontrolle dienen dann wie in den Beispielen gezeigt der Berechnung des Inhibitionsindex für die Substanzen, die als potentielle Inhibitoren zusammen mit dem Induktor TSP-1 in parallelen Untersuchungen eingesetzt werden. Eine Negativkontrolle zeigt das Fehlen sonstiger Induktoren an.

Ein positiver Inhibitionsindex zeigt dabei an, dass die entsprechende Substanz die Apoptose inhibiert. Ein negativer Inhibitionsindex zeigt dabei an, dass die verwendete Substanz die Apoptose induziert, d.h. die TSP-1 induzierte Apoptose verstärkt.

Native Peptide weisen allerdings oft eine geringe metabolische Stabilität gegenüber Peptidasen und eine relativ schlechte Bioverfügbarkeit auf.

Ausgehend von den oben gezeigten Peptiden kann der Fachmann ohne erfinderisches Zutun eine ganze Reihe von abgeleiteten Verbindungen entwickeln, die eine ähnliche oder gleichartige Wirkungsweise besitzen, und die u.a. auch Peptidmimetica genannt werden.

Als Peptidmimetica werden dabei für die Zwecke der vorliegenden Erfindung Verbindungen bezeichnet, welche die Struktur von Peptiden nachahmen und als Liganden in der Lage sind, die biologische Aktivität auf Rezeptor-/Enzymebene zu imitieren (Agonist) oder zu blockieren (Antagonist). Vor allem sollen die Peptidmimetica eine verbesserte Bioverfügbarkeit und eine verbesserte metabolische Stabilität aufweisen. Die Art der Mimetisierung kann von der leicht veränderten Ausgangsstruktur bis zum reinen Nichtpeptid reichen. Siehe beispielsweise A. Adang et al., Recl. Trav. Chim. Pays-Bas 113 (1994), 63-78.

Im Prinzip bieten sich folgende Möglichkeiten zur Mimetisierung/Derivatisierung einer Peptidstruktur an:

- Verwendung von D - statt L -Aminosäuren
- Modifizierung der Seitenkette von Aminosäuren
- Veränderung/Verlängerung der Peptidhauptkette
- Cyclisierung zur Konformationsstabilisierung
- Verwendung von Templaten, die eine bestimmte Sekundärstruktur erzwingen
- Verwendung eines nicht peptidischen Rückgrates, dass mit geeigneten Resten/Seitenketten die Struktur des Peptides nachahmt

Während die proteolytische Stabilität eines Peptids durch Austausch von L - gegen D -Aminosäuren gesteigert werden kann, führt die Modifikation der Seitenketten einer der Aminosäuren oft zu einer Verbesserung der Bindungseigenschaften des gesamten Peptids.

Bei der Veränderung des Peptidrückgrates erfolgt in der Regel ein Austausch einer Amidgruppe gegen amidähnliche Gruppierungen ( J. Gante, Angew. Chem. 106 (1994), 1780-1802). Durch diese Maßnahmen lassen sich sowohl die Bindungsaffinität als auch die metabolische Stabilität des nativen Peptids beeinflussen.

Durch die Cyclisierung eines linearen Peptids wird dessen Flexibilität und damit dessen globale Konformation festgelegt. Bei Fixierung der biologisch aktiven Konformation wird die Affinität des Peptids zum Rezeptor erhöht, da die Entropieabnahme bei der Bindung kleiner ist als bei der Bindung eines flexiblen, linearen Peptids. Zu diesem Zweck werden Aminosäureseitenketten, die nicht an der Rezeptorerkennung beteiligt sind, miteinander oder mit dem Peptidgerüst verknüpft.

Die Sekundärstruktur des Peptids spielt eine entscheidende Rolle für die molekulare Erkennung des Rezeptors. Neben $\alpha$-Helix und $\beta$-Faltblatt sind sogenannte Turns als Wendepunkte in der Peptidkette wichtige Konformationselemente. Der Ersatz dieser Struktureinheiten durch einen Baustein, welcher nach dem Einfügen in ein Peptid eine definierte Sekundärstruktur stabilisiert, hat zum Konzept des Sekundärstrukturmimeticum geführt.

Auch die Wasserlöslichkeit der Peptide kann beispielsweise durch Einführung von S- und C- Glycopeptid Derivaten erhöht werden. Weitere Maßnahmen können beispielsweise die PEGylierung der Peptide sein.

Auch die Lipophilie der Hexapeptide kann erhöht werden, indem beispielsweise Phenylalanine an die Peptidsequenz angehängt werden.

Die Cyclisierung bzw. die N-terminale Modifikation von Peptiden wird z.B. von Borchard, Journal of controlled Release 62 (1999), 231 -238 und von Blackwell et al., J. Org. Chem. 10 (2001), 5291-302 beschrieben.

Es ist daher klar, dass der Fachmann ausgehend von dem durch die vorliegende Erfindung vermittelte Wissen mit Leichtigkeit zu einer ganzen Reihe von abgeleiteten Peptidmimetika gelangen kann, die jedoch alle von der vorliegenden Erfindung mitumfässt sind.

Unter einem weiteren bevorzugten Gesichtspunkt stellt die vorliegende Erfindung daher auch Peptidmimetica zur Verfügung, die aus den SEQ ID NOs 12, 18 oder 19 abgeleitetet sind, sowie Substanzen, die solche Peptidmimetica umfassen. Insbesondere stellt die vorliegende Erfindung unter einem weiteren bevorzugten Gesichtspunkt die Verwendung von aus den SEQ ID NOs 12,18 oder 19 abgeleiteten Peptidmimetica und solche Peptidmimetica umfassenden Substanzen zur Herstellung eines Medikamentes zur Behandlung von Krebs zur Verfügung.

Unter einem weiteren bevorzugten Gesichtspunkt der vorliegenden Erfindung können diese Peptide bzw. entsprechend derivatisierte Peptide auch als Diagnostika eingesetzt werden. Wie von den Erfindern gezeigt wurde, binden die erfindungsgemäßen Substanzen, bevorzugt die Peptide der SEQ ID NOs 12,18 oder 19, an apoptotische Zellen, und lassen sich daher hervorragend als Diagnosetool einsetzen, so zum Beispiel um apoptotische Zellen im Bereich arteriosklerotischer Läsionen und damit die Lösionen überhaupt nachzuweisen Dazu werden die Substanzen markiert. Für diese Markierung sind dem Fachmann eine Vielzahl von Methoden bekannt, die je nach Einsatzzweck ausgewählt werden.

Zum Beispiel sind in den US-Patentschriften US 4,479,930 (Hnatowich), US 4,652,440 (Paik et al.) und US 4,668,503 (Hnatowich) geeignete Methoden beschrieben.

Für die Zwecke der vorliegenden Erfindung wird daher unter "markierte erfindungsgemäße Substanz" jede erfindungsgemäße Substanz verstanden, die eine im Stand der Technik vorbekannte und standardmäßig für die Markierung von Peptiden verwendete Markierungssubstanzen wie allgemein Radioisotope wie beispielsweise Rhenium oder Technetium, aber auch Enzyme, Enzymsubstrate, Antikörper, Epitope für die Erkennung über spezifische Antikörper/-fragmente usw. Der Fachmann wird die obige Aufzählung zwanglos als beispielhaft und nicht abschließend erkennen.

Sollen die erfindungsgemäßen Substanzen als Diagnosetools verwendet werden, kann daher jede im Stand der Technik vorbekannte Markierungsmethode verwendet werden.

Erfindungsgemäße Substanzen, die aktive Bestandteile einer pharmazeutischen Zubereitung sind, werden im Allgemeinen in einem pharmazeutisch annehmbaren Träger gelöst. Beispiele für pharmazeutisch annehmbare Träger können Pufferlösungen wie Phosphatpuffer oder Citratpuffer sein. Zur Aufrechterhaltung der Aktivität der Peptide können auch Reagenzien zugesetzt werden, die pharmazeutisch annehmbar sind und beispielsweise ein reduzierendes Milieu in der pharmazeutischen Zubereitung aufrechterhalten.

Die spezifische Dosierung und Posologie ist für jeden Patienten von einer Anzahl von Faktoren abhängig, einschließlich der Aktivität der verwendeten spezifischen Verbindungen, dem Alter des Patienten, dem Körpergewicht, dem allgemeinen Gesundheitszustand, dem Geschlecht, der Ernährung, der Zeit der Verabreichung, dem Weg der Verabreichung, der Exkretionsrate, der Verbindung mit anderen Arzneimitteln und der Schwere der einzelnen Erkrankung, für die die Therapie angewandt wird. Sie wird von einem Arzt in Abhängigkeit von diesen Faktoren ermittelt.

Peptidmedikamente werden üblicherweise parenteral verabreicht, z.B. durch ein Inhalationsspray, rektal, durch subkutane, intravenöse, intramuskuläre, intraartikuläre und intrathecale Injektions- und Infusionstechniken, oder äußerlich in pharmazeutischen Formulierungen, welche konventionelle, pharmazeutisch annehmbare Träger, Adjuvantien und Vehikel enthalten. Je nach Art der identifizierten Substanz kommen auch andere Verabreichungswege in Betracht, z.B. oral. Bei der Wundheilung werden die erfindungsgemäßen Identifikate bevorzugt in Form von Salben oder Puder verabreicht.

Die Erfindung stellt ebenso pharmazeutische Zusammensetzungen bereit, die eine wirksame Menge einer apoptotisch wirksamen Substanz, bevorzugt eines Peptids, Proteins oder Peptidmimeticums in Kombination mit einem konventionellen pharmazeutischen Träger umfassen. Ein pharmazeutischer Träger ist z.B. ein fester oder flüssiger Füllstoff, ein Verkapselungsmaterial oder ein Lösungsmittel. Beispiele für Materialien, die als pharmazeutische Träger dienen können, sind Zucker wie Lactose, Glucose und Saccharose; Stärke wie Maisstärke und Kartoffelstärke; Cellulose und deren Derivate wie Natriumcarboxymethylcellulose, Ethylcellulose und Celluloseacetat; pulverisierter Tragacanth; Malz; Gelatine; Talg; Arzneimittelträger wie Kakaobutter und Zäpfchenwachse; Öle wie Erdnussöl, Baumwollsamenöl, Färberdistelöl, Sesamöl, Olivenöl, Maisöl und Sojabohnenöl; Polyole wie Propylenglykol, Glycerin, Sorbitol, Mannitol und Polyethylenglykol; Ester wie Ethyloleat und Ethyllaureat; Agar; Puffermittel wie Magnesiumhydroxid und Aluminiumhydroxid; Alginsäure; Pyrogen-freies Wasser; isotonische Salzlösung; Ringers Lösung, Ethylalkohol und Phosphatpufferlösungen wie auch andere nichttoxische kompatible Substanzen, die in pharmazeutischen Formulierungen verwendet werden. Benetzungsmittel, Emulgatoren und Gleitmittel wie Natriumlaurylsulfat und Magnesiumstearat, wie auch Färbemittel, Beschichtungsmittel sowie Parfümierungsmittel und Konservierungsstoffe können ebenso in den Zubereitungen vorhanden sein, entsprechend den Anforderungen des Galenikers. Die Menge des aktiven Wirkstoffes, der mit den Trägermaterialien kombiniert wird, um eine Einzeldosis zu produzieren, wird abhängig von dem behandelten Patienten und der speziellen Methode der Verabreichung variieren.

Pharmazeurisch annehmbare Salze der erfindungsgemäßen Substanzen, bevorzugt Peptide, Proteine oder Peptidmimetica können auf gut bekanntem Weg, beispielsweise durch Lösen der erfindungsgemäßen Verbindungen in der entsprechenden, verdünnten Säure oder Base, z.B. Salzsäure oder Natronlauge, und anschließendem Gefriertrocknen hergestellt werden. Metallsalze können erhalten werden durch Lösen der erfindungsgemäßen Verbindungen in Lösungen, die das entsprechende Ion enthalten, und anschließendem Isolieren der Verbindung über HPLC oder Gelpermeationsverfahren.

Die folgenden Beispiele erläutern die Erfindung näher:

**BEISPIEL 1:** Kultivierung von humanen Endothelzellen aus Nabelschnur-Venen (HUVEC)

Lösungen (steril):

Kulturmedium: IF-Basalmedium + 15% (v/v) NCS, 5 $\mu$g/ml Transferrin, 5 $\mu$g/ml Heparin, 0,7 $\mu$g/ml FGF, 2 mM L-Glutamin [IF-Basalmedium: 1:1-Mischung aus Iscove's Modifiziertem Dulbecco's Medium (IMDM) und Ham's F12, beide von Life Technologies, Paisley (England)]
NCS: Serum neugeborener Kälber (Sebak, Aidenbach)
FGF: Fibroblastenwachstumsfaktor (eigene Herstellung, gereinigt aus Schweinehirn)

Materialien :

Zellkulturgefäße, gelatiniert

Durchführung,

Die Kultivierung von HUVEC erfolgt in gelatinebeschichteten Kulturgefäßen bei 37°C, 5% $CO_2$ und Wasserdampf-gesättigter Atmosphäre. Das Kulturmedium wird alle 2-3 Tage gewechselt; bei Konfluenz werden die Zellen mit einer Teilungsrate von 1:3 bis 1:5 passagiert. HUVEC wachsen streng kontaktinhibiert und bilden einschichtige Zellrasen mit der typischen Kopfsteinpflastermorphologie. Bei Konfluenz erreichen die Kulturen Zelldichten von 4-9 x $10^4$ Zellen/cm$^2$. Für Apoptoseuntersuchungen werden ausschließlich HUVEC-Kulturen der Passagen 1-4 verwendet.

*Beschichtung von Kulturgefäßen:*

Lösungen (steril) :

Gelatinelösung, 1% (w/v) in Milli-Q-Wasser
1 g Gelatine (zellkulturgetestet) in 100 ml Milli-Q-Wasser suspendieren, durch Autoklavieren für 20 min bei 121 °C und 2 bar lösen und bei Raumtemperatur lagern.
PBS (140 mM NaCl, 3 mM KCl, 8 mM $Na_2HPO_4$, 1,5 mM $KH_2PO_4$)
8 g/l NaCl
0,2 g/l KCl
1,44 g/l $Na_2HPO_4$ x 2 $H_2O$
0,2 g/l $KH_2PO_4$

Die Salze in einem entsprechenden Volumen Milli-Q-Wasser lösen, 20 min bei 121 °C und 2 bar autoklavieren und bei Raumtemperatur lagern. Der pH-Wert wird überprüft und liegt zwischen 7,2 und 7,4.

Materialien :

Zellkulturgefäße

Durchführung:

Für die Kultivierung adhärent wachsender Zellen werden Kulturgefäße mit Gelatine beschichtet. Der Boden der Zellkulturgefäße wird mit steriler Gelatinelösung bedeckt, die Zellkulturgefäße werden 15 min bei Raumtemperatur belassen. Die Gelatinelösung wird abgesaugt, die Zellkulturgefäße werden einmal mit PBS gewaschen und können so verwendet werden.

*Subkultivierung adhärenter Zellen*

Lösungen (steril):

PBS
Trypsin/EDTA-Lösung
0,05% (w/v) Trypsin und 0,02% (w/v)EDTA in PBS auflösen und sterilfiltrieren.

Materialien:

Zellkulturgefäße, gelatiniert

Durchführung:

Die Zellen werden mit Trypsin/EDTA-Lösung von der Kulturfläche abgelöst. Das Kulturmedium wird abgesaugt, der Boden des Kulturgefäßes kurz mit PBS gewaschen und mit Trypsin/EDTA-Lösung (~1 ml für eine 25 cm$^2$-Kulturflasche) bedeckt. Die Enzymlösung wird sofort wieder abgesaugt, so dass ein dünner Flüssigkeitsfilm auf den Zellen verbleibt. Die Zellen werden 1-10 min bei Raumtemperatur belassen und das Ablösen der Zellen unter dem Mikroskop verfolgt. Das Ablösen der Zellen kann durch sanftes Aufschlagen des Kulturgefäßes auf der Kante beschleunigt werden. Die Zellen werden in frischem Kulturmedium aufgenommen, eventuell gezählt und in neue Kulturgefäße ausgesät.

**BEISPIEL 2:** Bestimmung der Apoptoserate durch Färbung apoptotischer Zellen mit DAPI

DAPI gehört zur Indolfarbstoffgruppe und ist ein selektiver DNS-Farbstoff. Der Farbstoff wird bei 340-360 nm angeregt, und das Emissionsmaximum liegt bei 480 nm. Er wird für Apoptoseuntersuchungen eingesetzt [ vgl. Cohen et al., Immunology Today, 14, No. 3, 126-130 (1993)].

*Morphologische Auswertung*

Lösungen:

PBS
Formaldehydlösung
4% (v/v) Formaldehyd in PBS
DAPI-Lösung (Molecular Probes, Leiden, Niederlande)
2 μg/ml DAPI in Methanol

Materialien:

Petrischale (35 mm) mit Zellen in Kultur

Durchführung:

Der Kulturüberstand einer Petrischale wird abgesaugt, der Zellrasen wird 15 Minuten mit 1 ml Formaldehydlösung auf Eis fixiert, zweimal mit 2 ml PBS gewaschen, für 15 Minuten mit 0,5 ml DAPI-Lösung versetzt, mit PBS gewaschen und unter dem Fluoreszenzmikroskop ausgewertet. Es wird mit dem UV-Filtersatz und einem 20 x oder 40 x Objektiv gearbeitet. 500-1000 Zellen werden zufällig ausgewählt und die Zellen mit apoptotischen Kernen gezählt.

Der Apoptose-Index wird nach folgender Formel berechnet:

$$\text{Apoptose-Index [\%] = Anzahl apoptotischer Zellen/Gesamtzellzahl x 100}$$

*Durchflußcytometrie:*

Lösungen:

> PBS
> Medium
> Ethanol (p.a.) eisgekühlt (-20°C)
> DAPI-Puffer
> DAPI-Stammlösung
> DAPI-Färbelösung

Durchführung:

Der Kulturüberstand wird abgesaugt, und die Zellen werden, ohne sie mit PBS zu waschen, trypsiniert. Die Zellsuspension wird in Medium aufgenommen, gezählt, bei 800 x g für 5 Minuten zentrifugiert, das Sediment in 0,5 ml IF resuspendiert und in 1,5 ml eiskaltes Ethanol getropft. Die Suspension wird über Nacht bei - 20°C gelagert. Nach erneuter Zentrifugation und Resuspendierung des Sediments in 2 ml PBS folgt eine halbstündige Inkubation bei 37°C, eine weitere Zentrifugation, Resuspendierung des Sediments in 5 ml DAPI-Lösung und Auszählung im Durchflußcytometer bei einer Zählrate von 50-300 Ereignissen pro Sekunde. Die erhaltene Auftragung zeigt einen hohen Gipfel an Zellen in der $G_1$-Phase des Zellzyklus, gefolgt von einer Fraktion von Zellen in der S-Phase (mittlere Fluoreszenzintensitäten) und einem letzten Gipfel hoher Fluoreszenzintensitäten, der die Zellen in der $G_2$-Phase repräsentiert. Apoptotische Zellen erscheinen, bedingt durch die abnehmende absolute DNS-Menge pro Zelle, in einem Sub-$G_1$-Gipfel [Darzynklewicz Z. et al., Cytometry, 13, 795-808 (1992); Zamai L. et al., Cytometry, 14, 891-897 (1993)]. Dies zeigt das Auftreten einer Apoptose unter den gewählten Bedingungen.

**BEISPIEL 3:** Induktion der Apoptose und Testsystem für apoptotisch wirksame Peptide bzw. Proteine bei kultivierten Endothelzellen

Die Zellen werden wie in Beispiel 1 beschrieben kultiviert. Nach dem Erreichen der vollständigen Konfluenz werden die Zellen für den Test eingesetzt. Zunächst wird Thrombospondin 1 (1µg/ml) zu frischem Medium gegeben und mit der Wirkung von frischem Medium auf die Apoptoserate von HUVEC verglichen. Tabelle 1 zeigt, dass die Zugabe von Thrombospondin zu einer signifikanten Erhöhung der Apoptoserate führt. Die beobachtete Apoptose bei Zugabe von frischem Medium wird durch das während der Dauer des Experiments sekretierte Thrombospondin induziert..

**Tabelle 1: Induktion der Apoptose bei HUVEC mit TSP-1**

| Kulturmedium | Apoptoserate (%) nach 24 h |
|---|---|
| frisches Medium | $0,9 \pm 0,1$ |
| frisch + TSP-1 (1 µg/ml) | $3,0 \pm 0,4$ |

**BEISPIEL 4**: Peptidsynthese

Die verwendeten Peptide wurden durch das NMI (Naturwissenschaftliches und Medizinisches Institut an der Uni Tübingen, Reutlingen, Deutschland) nach Vorgaben der CytoTools synthetisiert.

**BEISPIEL 5**: Identifikation von apoptotisch wirksamen Hexamer-Peptiden mit Hilfe des erfindungsgemäßen Verfahrens

Die Zellen werden wie in Beispiel 1 beschrieben kultiviert. Die Zellen werden in die entsprechenden Kulturgefäße (z.B. 24-Lochplatte/0,5 ml pro Kavität) ausgesät und nach dem Erreichen der vollständigen Konfluenz für den Test eingesetzt. Die Zellen werden mit neuem Medium versorgt:

(a) frisches Kulturmedium **[Basisrate der Apoptose],**

(b) frisches Medium mit 1 μg/ml TSP-1 **[Apoptose-induzierende Substanz; Kontrolle],**

(1) Medium (b) + Peptid der SEQ ID NO 12, 1mM

(r) Medium (b) + Peptid der SEQ ID NO 18, 1mM

(s) Medium (b) + Peptid der SEQ ID NO 19, 1mM

Nach 24 h Inkubation unter Kulturbedingungen (Beispiel 1) werden die Zellen fixiert, mit DAPI gefärbt und morphologisch unter dem Fluoreszenzmikroskop untersucht bzw. durchflußcytometrisch untersucht. Die apoptischen Zellen und die Gesamtzellzahl werden bestimmt und der Apoptoseindex berechnet (Prozent apoptischer Zellen). Die Daten von 3 unabhängigen Experimenten mit der Angabe der Mittelwerte und der Standardabweichung sind in Tabelle 2 angegeben. Inhibitorisch wirksame Peptide weisen erfindungsgemäß einen positiven Inhibitionsindex auf, während induktorische Peptide erfindungsgemäß einen negativen Inhbtionsindex aufweisen.

**Tabelle 2:** Es werden folgende Peptide getestet:

| SEQ ID NO | Aminosäure-Sequenz | Apoptose index [%] | Inhibitions -index [%] * |
|---|---|---|---|
| K | Kontrolle | 3,28 ± 0,55 | |
| (12) | M-V-V-Y-F-R | 4,40±0,53 | -34,1 ± 6,2 |
| (18) | m-v-v-y-f-r | 14,6±0,75 | -345,1±5,1 |
| (19) | m-v-v-y-a-r | 11,4±0,63 | -247,1±5,4 |
| * positiver Inhibitionsindex = Substanz inhibiert; negativer Inhibitionsindex = Substanz induziert; | | | |

Erfindungsgemäß wird der Inhibitionsindex wie folgt errechnet:

$$\text{- Inhibitionsindex [\%] = (gemessener Apoptoseindex} * 100/\text{Apoptoseindexkontrolle}) - 100$$

**Patentansprüche**

1. Apoptotisch wirksame Substanz, **dadurch gekennzeichnet, dass** sie mindestens einen Peptidanteil mit einer der in den SEQ ID NOs 12, 18 und 19 dargestellten Aminosäuresequenzen umfasst und Apoptose-induzierend wirksam ist.

2. Apoptotisch wirksame Substanz nach Anspruch 1, **dadurch gekennzeichnet, dass** die apoptotisch wirksame Substanz ein Peptid ist, das eine Kettenlänge von < 100 Aminosäuren aufweist.

3. Apoptotisch wirksame Substanz nach Anspruch 2, **dadurch gekennzeichnet, dass** die apoptotisch wirksame Substanz ein Peptid ist, das eine Kettenlänge von < 50 Aminosäuren aufweist.

4. Pharmazeutische Zusammensetzung, umfassend mindestens eine Substanz nach einem der Ansprüche 1 bis 3 oder ein pharmazeutisch annehmbares Salz dieser Substanz und einen pharmazeutisch annehmbaren Träger.

5. Verwendung einer Substanz nach einem der Ansprüche 1 bis 3 oder eines pharmazeutisch annehmbaren Salzes hierzu zur Herstellung eines Medikamentes.

6. Verwendung einer Substanz nach Anspruch 5 oder eines pharmazeutisch annehmbaren Salzes hierzu zur Herstellung eines Medikamentes zur Behandlung von Krebs.

7. Medikament zur Behandlung von Krebs, umfassend mindestens eine Substanz nach einem der Ansprüche 1 bis 3 oder ein pharmazeutisch annehmbares Salz der apoptotisch wirksamen Substanz und einen pharmazeutisch annehmbaren Träger.

8. Substanz nach mindestens einem der Ansprüche 1 bis 3, welche markiert ist.

**Claims**

1. An apoptotically active substance, **characterised in that** it comprises at least one peptide portion having one of the amino acid sequences shown in SEQ ID NOs 12, 18 and 19 and induces apoptosis.

2. The apoptotically active substance according to Claim 1, **characterised in that** the apoptotically active substance is a peptide that has a chain length of < 100 amino acids.

3. The apoptotically active substance according to Claim 2, **characterised in that** the apoptotically active substance is a peptide that has a chain length of < 50 amino acids.

4. A pharmaceutical composition, comprising at least one substance according to one of Claims 1 to 3 or a pharmaceutically acceptable salt of this substance and a pharmaceutically acceptable carrier.

5. Use of a substance according to one of Claims 1 to 3 or of a pharmaceutically acceptable salt for preparation of a drug.

6. The use of a substance according to Claim 5 or of a pharmaceutically acceptable salt for preparation of a drug for the treatment of cancer.

7. A drug for the treatment of cancer, comprising at least one substance according to one of Claims 1 to 3 or a pharmaceutically acceptable salt of the apoptotically active substance and a pharmaceutically acceptable carrier.

8. The substance according to at least one of Claims 1 to 3, which substance is labelled.


**Revendications**

1. Substance active sur l'apoptose, **caractérisée en ce qu'**elle comprend au moins une partie de peptide avec l'une des séquences d'acides aminés représentées dans les SEQ ID NO 12, 18 et 19 et a un effet sur l'apoptose.

2. Substance active sur l'apoptose selon la revendication 1, **caractérisée en ce que** la substance active sur l'apoptose est un peptide qui présente une longueur de chaîne < 100 acides aminés.

3. Substance active sur l'apoptose selon la revendication 1, **caractérisée en ce que** la substance active sur l'apoptose est un peptide qui présente une longueur de chaîne < 50 acides aminés.

4. Composition pharmaceutique comprenant au moins une substance selon l'une des revendications 1 à 3, ou un sel pharmaceutiquement acceptable de cette substance et un support pharmaceutiquement acceptable.

5. Utilisation d'une substance selon l'une des revendications 1 à 3, ou d'un sel pharmaceutiquement acceptable de celle-ci, pour la fabrication d'un médicament.

6. Utilisation d'une substance selon la revendication 5, ou d'un sel pharmaceutiquement acceptable de celle-ci, pour la fabrication d'un médicament pour le traitement du cancer.

7. Médicament pour le traitement du cancer comprenant au moins une substance selon l'une des revendications 1 à 3, ou un sel pharmaceutiquement acceptable de la substance active sur l'apoptose et un support pharmaceutiquement acceptable.

8. Substance selon au moins l'une des revendications 1 à 3, laquelle est marquée.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4479930 A, Hnatowich
- US 4652440 A, Paik
- US 4668503 A, Hnatowich

**In der Beschreibung aufgeführte Nicht-Patentliteratur**

- **A. ADANG et al.** *Recl. Trav. Chim. Pays-Bas,* 1994, vol. 113, 63-78
- **J. GANTE.** *Angew. Chem.,* 1994, vol. 106, 1780-1802
- **VON BORCHARD.** *Journal of controlled Release,* 1999, vol. 62, 231-238
- **VON BLACKWELL et al.** *J. Org. Chem.,* 2001, vol. 10, 5291-302
- **COHEN et al.** *Immunology Today,* 1993, vol. 14 (3), 126-130
- **DARZYNKLEWICZ Z. et al.** *Cytometry,* 1992, vol. 13, 795-808
- **ZAMAI L. et al.** *Cytometry,* 1993, vol. 14, 891-897